# EUROPÄISCHE PATENTANMELDUNG

(11) **EP 2 452 639 A1**
(43) Veröffentlichungstag der Anmeldung: **16.05.2012**
(21) Anmeldenummer: 11188539.8
(22) Anmeldetag: 10.11.2011
(51) Int. Cl.: A61B 17/34

(54) **Chirugisches Dichtelement, chirurgische Dichtung sowie chirurgisches Abdichtungssystem**

(30) Priorität: 11.11.2010 DE 102010060490
(71) Anmelder: Aesculap AG, 78532 Tuttlingen (DE)
(72) Erfinder: Mayenberger, Rupert, 78239 Rielasingen (DE); Schweitzer, Tom, 78532 Tuttlingen (DE)
(74) Vertreter: Hoeger, Stellrecht & Partner Patentanwälte

(57) **Zusammenfassung**

Um ein chirurgisches Dichtelement, insbesondere für einen Trokar und/oder zum Abdichten von Schäften langgestreckter chirurgischer Instrumente beim Einführen in einen menschlichen oder tierischen Körper, welches eine Längsachse definiert und eine im Durchmesser veränderliche und quer oder im Wesentlichen quer zur Längsachse orientierte Öffnung aufweist, durch die ein Instrument einführbar ist, und eine ringförmig geschlossene, flexible Wand umfasst, welche Wand einen ersten und einen zweiten, jeweils in sich geschlossenen Rand aufweist, wobei der erste Rand die Öffnung begrenzt, so zu verbessern, dass stets eine optimale Abdichtung gewährleistet ist, wird vorgeschlagen, dass die Wand im Bereich zwischen dem ersten und dem zweiten Rand ein mindestens abschnittsweise umlaufendes Stützelement trägt.

Ferner werden eine verbesserte chirurgische Dichtung sowie eine verbessertes chirurgisches Abdichtungssystem vorgeschlagen.

## Beschreibung

Die vorliegende Erfindung betrifft ein chirurgisches Dichtelement, insbesondere für einen Trokar und/oder zum Abdichten von Schäften langgestreckter chirurgischer Instrumente beim Einführen in einen menschlichen oder tierischen Körper, welches eine Längsachse definiert und eine im Durchmesser veränderliche und quer oder im Wesentlichen quer zur Längsachse orientierte Öffnung aufweist, durch die ein Instrument einführbar ist, und eine ringförmig geschlossene, flexible Wand umfasst, welche Wand einen ersten und einen zweiten, jeweils in sich geschlossenen Rand aufweist, wobei der erste Rand die Öffnung begrenzt.

Ferner betrifft die vorliegende Erfindung eine chirurgische Dichtung, insbesondere für einen Trokar und/oder zum Abdichten von Schäften langgestreckter chirurgischer Instrumente beim Einführen in einen menschlichen oder tierischen Körper, wobei die Dichtung ein erstes Dichtelement umfasst, welche eine Längsachse definiert, mit einer im Durchmesser veränderlichen und quer oder im Wesentlichen quer zur Längsachse orientieren Öffnung versehen ist, durch die ein Instrument eingeführt werden kann, und eine erste ringförmig geschlossene, flexible Wand umfasst, wobei die erste Wand einen ersten und einen zweiten, jeweils in sich geschlossenen Rand aufweist und wobei der erste Rand die Öffnung begrenzt, wobei die erste Wand in einer ersten Dichtstellung derart wellenförmig ohne Knicke mit in Richtung auf den ersten Rand hin verlaufenden Faltlinien gefaltet ist, dass der erste Rand eine Wellenlinie definiert, welche vollständig auf einer Zylinderfläche liegt, wobei ein zweites, identisch oder im Wesentlichen identisch mit dem ersten Dichtelement ausgebildetes Dichtelement mit einer zweiten Wand, die auch einen ersten und einen zweiten Rand aufweist, vorgesehen ist und wobei der erste Rand des ersten Dichtelements mit dem ersten Rand des zweiten Dichtelements verbunden ist, so dass in der ersten Dichtstellung ein Wellenberg einer durch den ersten Rand des ersten Dichtelements definierten ersten Wellenlinie in ein Wellental einer durch den ersten Rand des zweiten Dichtelements definierten Wellenlinie eingreift.

Des Weiteren betrifft die vorliegende Erfindung ein chirurgisches Abdichtungssystem zum Einführen chirurgischer Instrumente in einen menschlichen oder tierischen Körper, vorzugsweise längs einer vom Abdichtungssystem definierten Längsachse, umfassend einen eine Einführöffnung aufweisenden Trokar und ein die Einführöffnung mindestens teilweise verschließendes, eine Öffnung aufweisendes chirurgisches Dichtelement zum Abdichten der Einführöffnung beim Einführen eines Instruments in die Öffnung.

Ein chirurgisches Dichtelement, eine chirurgische Dichtung und ein chirurgisches Abdichtungssystem der eingangs beschriebenen Art sind beispielsweise aus der WO 2007/110371 A1 bekannt. Derartige Dichtelemente beziehungsweise Dichtungen können insbesondere als Universaldichtungen eingesetzt werden, die den gesamten Dichtungsbereich für Instrumentenschäfte mit Durchmessern von 5 mm bis 13 mm abdecken. Um das chirurgische Dichtelement beziehungsweise die chirurgische Dichtung vor spitzen oder scharfen Instrumentenspitzen zu schützen, ist es zudem bekannt, eine an das Dichtelement beziehungsweise die Dichtung angepasste Schutzvorrichtung vorzusehen, wie sie beispielsweise aus der EP 2 143 393 A1 bekannt ist.

Nachteilig bei den bekannten Dichtelementen und Dichtungen ist jedoch, dass beim Einsatz eines mit einem solchen Dichtelement oder eine solchen Dichtung ausgestatteten Trokars Instrumente mit einem sehr kleinen Durchmesser in dem im Durchmesser deutlich größeren Trokarschaft seitlich ausgelenkt werden können. Bei sehr starker seitlicher Auslenkung können die Rückstellkräfte des Dichtelements dazu führen, dass der erste Rand, der optional eine Dichtlippe tragen kann, einseitig aufgezogen wird, was zu einer unerwünschten Undichtigkeit führt. Ferner werden beim Einführen von Instrumenten mit sehr großen Durchmessern, zum Beispiel eines Obturators des Trokars oder von Nahtklammergeräten, über einen längeren Zeitraum, Schutzelemente der Schutzvorrichtung, die insbesondere lamellenartig ausgebildet sein können, einer großen Biegebeanspruchung ausgesetzt. Da der rein elastische Dehnbereich von thermoplastischen Materialien beziehungsweise thermoplastischen Elastomermaterialien, aus denen die Schutzvorrichtung vorzugsweise hergestellt ist, eingeschränkt ist, kann es somit beim Einführen von Instrumenten mit sehr dicken Schäften zu plastischen Verformungen der Schutzvorrichtung im Bereich seiner Schutzelemente kommen. Durch diesen Effekt wird eine Undichtigkeit bei seitlicher Auslenkung eines eingeführten Instruments noch weiter verstärkt.

Es ist daher Aufgabe der vorliegenden Erfindung, ein chirurgisches Dichtelement, eine chirurgische Dichtung und ein chirurgisches Abdichtungssystem der eingangs beschriebenen Art so zu verbessern, dass stets eine optimale Abdichtung gewährleistet ist.

Diese Aufgabe wird bei einem chirurgischen Dichtelement der eingangs beschriebenen Art erfindungsgemäß dadurch gelöst, dass die Wand im Bereich zwischen dem ersten und dem zweiten Rand ein mindestens abschnittsweise umlaufendes Stützelement trägt.

Durch das Stützelement kann einerseits ein Auslenken der Öffnung des Dichtelements beim Einführen eines Instruments minimiert beziehungsweise ein Rückführen der Öffnung in die Grundstellung verbessert werden. Des Weiteren ermöglicht es das Stützelement, und zwar je nach Ausbildung der Schutzvorrichtung, Schutzelemente derselben in einer Grundstellung zu halten sowie deren Rückführung in die Grundstellung infolge einer Auslenkung durch Einführen eines Instruments zu erleichtern. Des Weiteren kann das Stützelement auch als Widerlager eine Verkippbewegung der Schutzvorrichtung in Richtung der Instrumentenauslenkung unterstützen. Insbesondere kann das Stützelement die Wider- oder Gegenlagerfunktion im Zusammenwirken mit einer oder mehreren Anschlagflächen an der Trokarhülse oder einer Halterung der Dichtung übernehmen. Es kann sich bei der Anschlagfläche um eine Ringfläche handeln, welche auf die Längsachse oder im Wesentlichen auf die Längsachse der Trokarhülse oder Halterung hin weist. Wird infolge einer seitlichen Auslenkung eines eingeführten Instruments das Dichtelement mit der ersten Wand im Bereich des Stützelements gegen die Anschlagfläche beziehungsweise die Anschlagflächen gedrückt, bewirkt eine weitere Auslenkung ein Verkippen des Dichtelements insgesamt längs der Längsachse um einen Drehpunkt, welcher im Wesentlichen durch den Kontakt zwischen Anschlagfläche und erster Wand im Bereich des Stützelements definiert wird. Dieses Prinzip ist besonders vorteilhaft bei Dichtelementen mit gefalteten Wänden, die auch als Faltdichtungen bezeichnet werden, welche bedingt durch ihre Faltung nur eine geringe Umfangsspannung im Bereich der Öffnung aufweisen. Undichtigkeiten bei seitlicher Auslenkung können so auf einfache Weise verhindert werden. Des Weiteren bietet das Stützelement eine verbesserte Zentrierfunktion beim Einführen von Instrumenten, wodurch gleichzeitig ein Beschädigungsrisiko für das Dichtelement verringert wird. Das Stützelement muss nicht zwingend eine in radialer Richtung definierte konstante Dicke aufweisen. Insbesondere kann es in Umfangsrichtung auch nur abschnittweise ausgebildet sein, um genau dort, wo Schutzelemente positioniert sind, diesen als Widerlager zu dienen. Vorzugsweise ist die erste Wand wellenförmig faltbar.

Vorteilhaft ist es, wenn das Stützelement in Richtung auf die Längsachse vorspringend an der Wand angeordnet oder ausgebildet ist. Auf diese Weise kann es als Anlage für Schutzelemente der Schutzvorrichtung dienen und gleichzeitig die Schutzelemente vom Dichtelement, das heißt von dessen Wand, in definierter Weise beabstandet halten.

Besonders einfach ausbilden lässt sich das Dichtelement, wenn das Stützelement in Form eines Vorsprungs ausgebildet ist.

Die Herstellung des Dichtelements lässt sich weiter vereinfachen, wenn das Stützelement und die Wand einstückig ausgebildet sind. Sie können wahlweise aus einem einzigen Material hergestellt sein oder aber auch aus unterschiedlichen Materialien.

Günstig ist es, wenn das Stützelement eine mindestens teilweise auf die Längsachse hin weisende Stützfläche für Schutzelemente einer Schutzvorrichtung des Dichtelements aufweist. Die Stützfläche kann insbesondere an die Form sowie die Position der Schutzelemente der Schutzvorrichtung angepasst sein, um so die Schutzelemente in definierter Weise stützen und gegebenenfalls wieder in ihre Grundstellung zurückbewegen zu können.

Um Beschädigungen der Schutzelemente möglichst zu vermeiden, ist es vorteilhaft, wenn die Stützfläche in Richtung auf die Längsachse hin konvex gewölbt ist. Insbesondere kann sie wulstartig ausgebildet sein, so dass sich die Schutzelemente beim Abstützen an der Stützfläche definiert verbiegen können, jedoch insbesondere nicht einer Kerbwirkung von beispielsweise einer scharfen Kante ausgesetzt sind. Durch die Form der Stützfläche lässt sich eine Verformung der Schutzelemente, insbesondere infolge einer Auslenkung eines eingeführten Instruments bezogen auf die Längsachse, in definierter Weise vorgeben und führen.

Die Herstellung des Dichtelements vereinfacht sich weiter, wenn das Stützelement in Form eines in sich geschlossenen Rings ausgebildet ist. Ein solcher Ring muss nicht zwangsläufig kreisförmig ausgebildet sein. Er kann beispielsweise eine sich an eine gefaltete Oberfläche der Wand angepasste oder sich an diese anpassende Kontur aufweisen. Durch die Form eines Rings können zudem auf einer Seite der Wand einwirkende Kräfte auf die gesamte Wand übertragen werden, so dass das gesamte Dichtelemente infolge einer Auslenkung mit ausgelenkt wird, was das Einführen des Instrumentenschafts durch die Öffnung erleichtert und somit das Risiko für eine Verletzung des Dichtelements minimiert.

Bei einer besonders bevorzugten Ausführungsform der Erfindung kann vorgesehen sein, dass ein Abstand des Stützelements von der Längsachse in einer Grundstellung des Dichtelements, in welcher die Öffnung einen minimalen Durchmesser aufweist, in Abhängigkeit eines Umfangswinkels bezogen auf die Längsachse variiert. Eine solche Ausgestaltung kann insbesondere dadurch realisiert werden, dass ein Vorsprung mit konstanter Dicke in radialer Richtung auf einer inneren Oberfläche einer gefalteten Wand des Dichtelements angeordnet wird. Eine senkrechte Projektion einer vom Stützelement definierten Durchtrittsöffnung auf eine zur Längsachse senkrechte Projektionsebene ist dann einer vom Dichtelement definierten Durchtrittsöffnung in einer das Stützelement durchsetzenden Schnittebene der Wand geometrisch ähnlich.

Vorzugsweise weist eine Abstandsfunktion des Abstands in Abhängigkeit des Umfangswinkels mindestens ein Minimum und mindestens ein Maximum auf. Je nach Anzahl der Falten eines knickfrei faltbaren Dichtelements können auch mehrere Minima und Maxima auftreten, beispielsweise 5, 10 oder noch mehr, insbesondere kann die Zahl der Minima und Maxima vorzugsweise jeweils in einem Bereich von 4 bis 20 liegen.

Zur Optimierung der Herstellung des Dichtelements ist es günstig, wenn das Stützelement an die Wand angespritzt ist, und zwar insbesondere dann, wenn das Dichtelement selbst durch aus einem Kunststoff gespritzt wird, kann im selben oder einem nachfolgenden Fertigungsschritt das Stützelement an die Wand angeformt werden. Das Anspritzen des Stützelements ist insbesondere dann vorteilhaft, wenn das Stützelement und die Wand des Dichtelements nicht aus demselben Material hergestellt werden. Insbesondere kann es vorteilhaft sein, wenn eine Dehnbarkeit des Stützelements größer ist als die der Wand.

Um auf einfache Weise direkt mit Schutzelementen in Kontakt kommen zu können, ist es vorteilhaft, wenn das Stützelement auf einer der Längsachse zugewandten Innenseite der Wand angeordnet oder ausgebildet ist.

Damit das Dichtelement nach Möglichkeit stets in einer definierten Form gehalten werden kann, ist es vorteilhaft, wenn das Stützelement aus einem elastisch verformbaren und/oder dehnbaren Material gebildet ist. Das Stützelement selbst kann dann aufgrund einer Verformung und der aufgrund der Verformung gespeicherten Kräfte wieder in seine Ursprungsform von selbst zurückgehen. In Abhängigkeit von der Positionierung und Anordnung an der Wand kann dabei auch infolge einer Rückführung des Stützelements von einer ausgelenkten oder gedehnten Stellung in seine Grundstellung zurück die Wand des Dichtelements auch wieder in ihre ursprüngliche Form zurückgeführt werden.

Die Herstellung des Stützelements wir besonders einfach, wenn es aus einem Kunststoff hergestellt ist. Beispielsweise lässt es sich so durch Spritzgießen oder Anspritzen an die Wand herstellen.

Besonders einfach und kostengünstig herstellen lässt sich das Dichtelement, wenn der Kunststoff ein Elastomer ist. Vorzugsweise enthält der Kunststoff Gummi, Silikon oder Gummi und/oder Silikon. Günstig ist es, wenn das Elastomer thermoplastisch ist oder thermoplastische Eigenschaften aufweist. Vorteilhaft sind auch Kunststoffe in Form thermoplastischer Elastomere.

Um eine besonders definierte Rückführ- und Mitnahmebewegung des Stützelements relativ zur Wand und mit dieser vorzugeben, ist es vorteilhaft, wenn das Stützelement ausgehend von einer Grundstellung, in welcher die Öffnung einen minimalen Durchmesser aufweist, beim Übergang in eine Einführstellung, in welcher die Öffnung einen maximalen Durchmesser aufweist, maximal um 25 % dehnbar ist. Vorzugsweise ist das Stützelement maximal um 10 bis 20 % dehnbar. Insbesondere kann die Dehnbarkeit so zu verstehen sein, dass beispielsweise ein Innendurchmesser des Stützelements im angegebenen Bereich durch entsprechende Dehnung aufgeweitet werden kann.

Günstigerweise ist das Stützelement in einer Grundstellung, in welcher die Öffnung einen minimalen Durchmesser aufweist, ungedehnt. Um dies zu erreichen ist es günstig, wenn das Stützelement an das Dichtelement angeformt oder angespritzt wird, wenn sich dieses in einer Stellung befindet, in welcher keine äußere Kräfte auf es einwirken.

Ferner kann es vorteilhaft sein, wenn die Wand in einer Dichtstellung derart wellenförmig ohne Knicke mit in Richtung auf den ersten Rand hin verlaufenden Faltlinien gefaltet ist, dass der erste Rand eine Wellenlinie definiert, welche vollständig auf einer Zylinderfläche liegt. Insbesondere kann die Zylinderfläche koaxial zur Längsachse des Dichtelements ausgerichtet sein. Durch die besondere Ausgestaltung der Wellenlinie ist eine optimale Abdichtung eines insbesondere in der Regel zylindrisch ausgebildeten Instrumentenschafts unabhängig von einem Durchmesser desselben gewährleistet. Dies ermöglicht insbesondere den Einsatz als Universaldichtelement für Trokare.

Günstig kann es ferner auch sein, wenn die Wellenlinie oberhalb einer senkrecht zur Längsachse verlaufenden Öffnungsebene der Öffnung Wellenberge und unterhalb der Öffnungsebene Wellentäler aufweist. Insbesondere kann die Zahl der Wellenberge und Wellentäler zur Zahl der Minima und Maxima der oben definierten Abstandsfunktion korrespondieren. In diesem Fall hängt sie also insbesondere von der Zahl der Faltungen der Wand ab.

Gemäß einer weiteren bevorzugten Ausführungsform der Erfindung kann ferner vorgesehen sein, dass die Wand in einer Vielzahl von Dichtstellungen mit jeweils unterschiedlichen Durchmessern der Öffnung in Richtung auf den ersten Rand hin derart wellenförmig ohne Knicke gefaltet ist, dass der erste Rand jeweils eine Wellenlinie definiert, welche vollständig auf einer Zylinderfläche liegt. Mit einem solchen Dichtelement ist es somit möglich, unabhängig von einem Durchmesser des Schafts eines einzuführenden Instruments eine optimale Abdichtung zu erreichen.

Die eingangs gestellte Aufgabe wird ferner bei einer chirurgischen Dichtung der eingangs beschriebenen Art erfindungsgemäß dadurch gelöst, dass die erste Wand im Bereich zwischen dem ersten und dem zweiten Rand ein mindestens abschnittsweise umlaufendes Stützelement trägt.

Eine solche Ausgestaltung hat die bereits oben im Zusammenhang mit dem erfindungsgemäß vorgeschlagenen Dichtelement dargelegten Vorteile. Wie beim Dichtelement kann das Stützelement vorzugsweise näher am zweiten Rand angeordnet sein als am ersten Rand, insbesondere kann das Stützelement auch direkt an den ersten Rand angrenzen. Bevorzugte Ausführungsformen sowohl des Dichtelements als auch der Dichtung sehen vor, dass zwischen dem zweiten Rand und dem Stützelement mindestens ein Ausgleichselement vorgesehen ist, um zusätzlich eine seitliche Auslenkung der Öffnung relativ zur Längsachse des Dichtelements beziehungsweise der Dichtung in der Grundstellung zu ermöglichen. Vorzugsweise sind die erste und/oder die zweite Wand wellenförmig faltbar.

Vorteilhaft ist es, wenn das erste und/oder das zweite Dichtelement in Form eines der oben beschriebenen Dichtelemente ausgebildet sind. Die chirurgische Dichtung weist dann ebenfalls die oben im Zusammenhang mit den bevorzugten Ausführungsformen von Dichtelementen beschriebenen Vorteile auf. Ferner sei angemerkt, dass vorzugsweise nur am ersten Dichtelement der Dichtung ein Stützelement vorgesehen ist.

Des Weiteren wird die eingangs gestellte Aufgabe bei einem chirurgischen Abdichtungssystem der eingangs beschriebenen Art erfindungsgemäß dadurch gelöst, dass das Dichtelement eines der oben beschriebenen Dichtelemente oder eine der oben beschriebenen Dichtungen ist.

Ein solches Abdichtungssystem mit einem der oben beschriebenen Dichtelemente oder einer der oben beschriebenen Dichtungen weist insbesondere die oben im Zusammenhang mit den bevorzugten Ausführungsformen der Dichtelemente und Dichtungen beschriebenen Vorteile auf.

Vorteilhaft ist es, wenn das chirurgische Abdichtungssystem eine Dichtelementhalterung umfasst, welche einerseits mit dem Trokar und andererseits mit dem chirurgischen Dichtelement oder der Dichtung kraft- und/oder formschlüssig verbindbar ist. Dies gestattet es, beispielsweise das Dichtelement oder die Dichtung zunächst mit der Dichtelementhalterung und die Dichtelementhalterung danach mit dem Trokar zu verbinden, beispielsweise einem proximalen Ende einer Trokarhülse des Trokars, welche beim Einführen vom Instrument in einen Patientenkörper aus dem Patientenkörper herausragt. Um das Dichtelement beziehungsweise die Dichtung beim Einführen von insbesondere scharfen oder spitzen Instrumentenspitzen in den Trokar nicht zu beschädigen, ist es vorteilhaft, wenn das Abdichtungssystem eine Schutzvorrichtung für das Dichtelement oder die Dichtung umfasst. Die Schutzvorrichtung kann insbesondere am Trokar, also an der Trokarhülse, oder aber alternativ auch direkt am Dichtelement oder der Dichtung angeordnet beziehungsweise mit dem jeweiligen Teil verbunden werden.

Gemäß einer bevorzugten Ausführungsform des Abdichtungssystems kann vorgesehen sein, dass die Schutzvorrichtung einen am Trokar oder an einem Teil desselben anordenbaren, ringförmigen oder im Wesentlichen ringförmigen und eine Durchbrechung definierenden Grundkörper mit mehreren in Umfangsrichtung angeordneten und parallel oder auf eine Längsachse der Schutzvorrichtung hin weisenden Schutzelementen umfasst, welche Schutzelemente im Wesentlichen in distaler Richtung weisende freie Enden aufweisen. Der Grundkörper kann auf einfache Weise am Trokar oder einem Teil desselben, beispielsweise dem Dichtelement oder der Dichtelementhalterung, festgelegt werden. Die Schutzelemente sind vorzugsweise lamellenförmig ausgebildet, wobei eine Breite der Lamellen längs ihrer Länge variieren kann. Aus Stabilitätsgründen ist vorzugsweise eine Dicke der Lamellen konstant. Die Schutzvorrichtung ist bevorzugt einstückig ausgebildet und aus einem Kunststoff hergestellt, welcher flexibel und vorzugsweise teilweise elastisch ist.

Vorzugsweise ist eine am Grundkörper angeordnete Verbindungseinrichtung zum Verbinden der Schutzvorrichtung mit dem chirurgischen Dichtelement oder dem chirurgischen Abdichtungssystem vorgesehen. Insbesondere dann, wenn die Schutzvorrichtung direkt mit dem Dichtelement verbindbar ist, kann eine definierte Positionierung der Schutzelemente relativ zur gefalteten Wand erreicht werden, so dass beispielsweise längere Schutzelemente zwischen Wellentäler der Wand des Dichtelements eingreifen, kürzere Schutzelemente dagegen an Wellenberge der Wand in einer Grundstellung derselben in Anlage oder Kontakt kommen können.

Günstigerweise liegen die Schutzelemente mindestens abschnittsweise in einer Grundstellung, in welcher die Öffnung einen minimalen Durchmesser aufweist, am Stützelement an. Insbesondere können Schutzelemente durch das Stützelement bereits etwas in Richtung auf die Längsachse vorgespannt werden, was das Einführen des Instruments in den Trokar erleichtert und zudem eine Zentrierwirkung der Schutzvorrichtung und auch des Dichtelements beziehungsweise der Dichtung verbessert. Zudem wird noch besser verhindert, dass eine Instrumentenspitze beim Einführen in den Trokar direkt mit dem Dichtelement in Kontakt treten und dieses beschädigen kann. Ferner kann so auch eine Verformung der Schutzelemente der Schutzvorrichtungen in definierter Weise vorgegeben werden, da sie sich quasi über das Stützelement nur in definierter Weise abrollen können. Eine Verformung der Schutzelemente findet dann insbesondere im Bereich des Stützelements statt, welches ein Widerlager für eine Bewegung beziehungsweise Verformung der Schutzelemente bildet.

Um eine Führung der Schutzvorrichtung, die am Dichtelement oder der Dichtung angeordnet ist, noch weiter zu verbessern, ist es vorteilhaft, wenn mindestens 80 % der Länge der Schutzelemente sich distalseitig des Stützelements erstreckt. Vorzugsweise erstrecken sich mindestens 60 % der Länge des Schutzelements distalseitig des Stützelements. Auf diese Weise kann eine sehr stabile Verbindung und Führung zwischen dem Dichtelement und der Schutzvorrichtung erreicht werden, wobei die sich distalseitig des Stützelements erstreckenden Abschnitte der Schutzelemente noch eine ausreichende Flexibilität aufweisen können, um das Dichtelement zu schützen und gleichzeitig das Instrument beim Einführen zu führen.

Damit die Schutzelemente das Dichtelement vor Einführen eines Instruments möglichst vollständig abdecken, ist es günstig, wenn in der Grundstellung die Schutzelemente durch das Stützelement in Richtung auf die Längsachse hin vorgespannt sind. Insbesondere kann so auch erreicht werden, dass eine von den Schutzelementen definierte Öffnung im Bereich der Öffnung des Dichtelements einen kleineren Innendurchmesser aufweist als die Öffnung des Dichtelements, so dass mit großer Wahrscheinlichkeit sichergestellt werden kann, dass ein Instrumentenschaft beim Einführen in den Trokar zunächst mit distalen Enden der Schutzelemente in Kontakt tritt und nicht mit dem Dichtelement.

Eine besonders gute Anpassung einer Kontur des Dichtelements kann erreicht werden, wenn die Schutzelemente in sich flexibel ausgebildet sind. Vorzugsweise weisen sie jedoch eine höhere, insbesondere eine deutlich höhere Steifigkeit auf als das Dichtelement.

Besonders vorteilhaft ist es, wenn das chirurgische Abdichtungssystem mindestens einen in Richtung auf die Längsachse oder im Wesentlichen auf die Längsachse hin wirkenden Anschlag umfasst, welcher das Stützelement in Umfangsrichtung mindestens abschnittsweise umgibt und in der Grundstellung von der das Stützelement von der Längsachse weg weisend begrenzenden Wand beabstandet ist. Vorzugsweise liegt ein Abstand in radialer Richtung zwischen dem mindestens einen Anschlag und der Wand beziehungsweise dem Stützelement in einem Bereich von etwa 1 mm bis etwa 4 mm. Dies hat insbesondere den Vorteil, dass ein optimales Zusammenwirken des mindestens einen Anschlags und des Stützelements in einem gewünschten Auslenkungsbereich sichergestellt werden kann. Denkbar wäre auch ein einziger, sich in Umfangsrichtung erstreckender Anschlag. Der mindestens eine Anschlag weist vorzugsweise eine in Richtung auf die Wand beziehungsweise das Stützelement hin weisende Anschlagfläche auf. Es kann sich bei der Anschlagfläche beispielsweise um eine Ringfläche handeln, welche auf die Längsachse oder im Wesentlichen auf die Längsachse der Trokarhülse oder Halterung hin weist. Die Ringfläche kann beispielsweise auch unterbrochen sein. Im Zusammenwirken mit dem mindestens einen Anschlag kann das Stützelement insbesondere als Widerlager eine Verkippbewegung der Schutzvorrichtung in Richtung der Instrumentenauslenkung unterstützen.

Günstig ist es, wenn der mindestens eine Anschlag an einer Dichtelementhalterung und/oder an einem das Dichtelement oder die chirurgische Dichtung umgebenden Gehäuse angeordnet oder ausgebildet ist. So kann insbesondere auf einfache Weise sichergestellt werden, dass beim Zusammenbau der mindestens eine Anschlag automatisch genau so positioniert wird, dass er optimal mit dem Stützelement zusammenwirken kann.

Die vorstehende Beschreibung umfasst somit insbesondere die nachfolgend in Form durchnummerierter Sätze definierte Ausführungsformen eines chirurgischen Dichtelements, einer chirurgischen Dichtung sowie eines chirurgischen Abdichtungssystems:
1. Chirurgisches Dichtelement (16), insbesondere für einen Trokar und/oder zum Abdichten von Schäften (170) langgestreckter chirurgischer Instrumente beim Einführen in einen menschlichen oder tierischen Körper, welches eine Längsachse (20) definiert und eine im Durchmesser veränderliche und quer oder im Wesentlichen quer zur Längsachse orientierte Öffnung (54) aufweist, durch die ein Instrument einführbar ist, und eine ringförmig geschlossene, flexible Wand (42) umfasst, welche Wand (42) einen ersten und einen zweiten, jeweils in sich geschlossenen Rand (58, 60) aufweist, wobei der erste Rand (58) die Öffnung (54) begrenzt, dadurch gekennzeichnet, dass die Wand (42) im Bereich zwischen dem ersten und dem zweiten Rand (58, 60) ein mindestens abschnittsweise umlaufendes Stützelement (62) trägt.
2. Chirurgisches Dichtelement nach Satz 1, dadurch gekennzeichnet, dass das Stützelement (62) in Richtung auf die Längsachse (20) vorspringend an der Wand (42) angeordnet oder ausgebildet ist.
3. Chirurgisches Dichtelement nach einem der voranstehenden Sätze, dadurch gekennzeichnet, dass das Stützelement (62) in Form eines Vorsprungs ausgebildet ist.
4. Chirurgisches Dichtelement nach einem der voranstehenden Sätze, dadurch gekennzeichnet, dass das Stützelement (62) und die Wand (42) einstückig ausgebildet sind.
5. Chirurgisches Dichtelement nach einem der voranstehenden Sätze, dadurch gekennzeichnet, dass das Stützelement (62) eine mindestens teilweise auf die Längsachse hin weisende Stützfläche (68, 70) für Schutzelemente (158, 160) einer Schutzvorrichtung (146) des Dichtelements (16) aufweist.
6. Chirurgisches Dichtelement nach Satz 5, dadurch gekennzeichnet, dass die Stützfläche (68, 70) in Richtung auf die Längsachse (20) hin konvex gewölbt ist.
7. Chirurgisches Dichtelement nach einem der voranstehenden Sätze, dadurch gekennzeichnet, dass das Stützelement (62) in Form eines in sich geschlossenen Rings ausgebildet ist.
8. Chirurgisches Dichtelement nach einem der voranstehenden Sätze, dadurch gekennzeichnet, dass ein Abstand des Stützelements (62) von der Längsachse (20) in einer Grundstellung des Dichtelements (16), in welcher die Öffnung (54) einen minimalen Durchmesser aufweist, in Abhängigkeit eines Umfangswinkels bezogen auf die Längsachse (20) variiert.
9. Chirurgisches Dichtelement nach Satz 8, dadurch gekennzeichnet, dass eine Abstandsfunktion des Abstands in Abhängigkeit des Umfangswinkels mindestens ein Minimum und mindestens ein Maximum aufweist.
10. Chirurgisches Dichtelement nach einem der voranstehenden Sätze, dadurch gekennzeichnet, dass das Stützelement (62) an die Wand (42) angespritzt ist.
11. Chirurgisches Dichtelement nach einem der voranstehenden Sätze, dadurch gekennzeichnet, dass das Stützelement (62) auf einer der Längsachse (20) zugewandten Innenseite (66) der Wand (42) angeordnet oder ausgebildet ist.
12. Chirurgisches Dichtelement nach einem der voranstehenden Sätze, dadurch gekennzeichnet, dass das Stützelement (62) aus einem elastisch verformbaren und/oder dehnbaren Material gebildet ist.
13. Chirurgisches Dichtelement nach einem der voranstehenden Sätze, dadurch gekennzeichnet, dass das Stützelement (62) aus einem Kunststoff hergestellt ist.
14. Chirurgisches Dichtelement nach Satz 13, dadurch gekennzeichnet, dass der Kunststoff ein Elastomer ist, vorzugsweise Gummi, Silikon oder Gummi und/oder Silikon enthält.
15. Chirurgisches Dichtelement nach einem der voranstehenden Sätze, dadurch gekennzeichnet, dass das Stützelement (62) ausgehend von einer Grundstellung, in welcher die Öffnung (54) einen minimalen Durchmesser aufweist, beim Übergang in eine Einführstellung, in welcher die Öffnung (54) einen maximalen Durchmesser aufweist, maximal um 100% dehnbar ist, vorzugsweise maximal um 10 bis 50%.
16. Chirurgisches Dichtelement nach einem der voranstehenden Sätze, dadurch gekennzeichnet, dass das Stützelement (62) in einer Grundstellung, in welcher die Öffnung (54) einen minimalen Durchmesser aufweist, ungedehnt ist.
17. Chirurgisches Dichtelement nach einem der voranstehenden Sätze, dadurch gekennzeichnet, dass die Wand (42) in einer Dichtstellung derart wellenförmig ohne Knicke mit in Richtung auf den ersten Rand (58) hin verlaufenden Faltlinien gefaltet ist, dass der erste Rand (58) eine Wellenlinie (44) definiert, welche vollständig auf einer Zylinderfläche liegt.
18. Chirurgisches Dichtelement nach Satz 17, dadurch gekennzeichnet, dass die Wellenlinie (44) oberhalb einer senkrecht zur Längsachse (20) verlaufenden Öffnungsebene (22) der Öffnung (54) Wellenberge (48) und unterhalb der Öffnungsebene (22) Wellentäler (50) aufweist.
19. Chirurgisches Dichtelement nach einem der voranstehenden Sätze, dadurch gekennzeichnet, dass die Wand (42) in einer Vielzahl von Dichtstellungen mit jeweils unterschiedlichen Durchmessern der Öffnung (54) in Richtung auf den ersten Rand (58) hin derart wellenförmig ohne Knicke gefaltet ist, dass der erste Rand (58) jeweils eine Wellenlinie (44) definiert, welche vollständig auf einer Zylinderfläche liegt.
20. Chirurgische Dichtung, insbesondere für einen Trokar und/oder zum Abdichten von Schäften (170) langgestreckter chirurgischer Instrumente beim Einführen in einen menschlichen oder tierischen Körper, wobei die Dichtung ein erstes Dichtelement (16) umfasst, welches eine Längsachse (20) definiert, mit einer im Durchmesser veränderlichen und quer oder im Wesentlichen quer zur Längsachse (20) orientierten Öffnung (54) versehen ist, durch die ein Instrument eingeführt werden kann, und eine erste ringförmig geschlossene, flexible Wand (42) umfasst, wobei die erste Wand (42) einen ersten und einen zweiten, jeweils in sich geschlossenen Rand (58, 60) aufweist und wobei der erste Rand (58) die Öffnung (54) begrenzt, wobei die erste Wand (42) in einer ersten Dichtstellung derart wellenförmig ohne Knicke mit in Richtung auf den ersten Rand (58) hin verlaufenden Faltlinien gefaltet ist, dass der erste Rand (58) eine Wellenlinie (44) definiert, welche vollständig auf einer Zylinderfläche liegt, wobei ein zweites, identisch oder im Wesentlichen identisch mit dem ersten Dichtelement (16) ausgebildetes Dichtelement (16) mit einer zweiten Wand (42), die auch einen ersten und zweiten Rand (58, 60) aufweist, vorgesehen ist und wobei der erste Rand (58) des ersten Dichtelements (16) mit dem ersten Rand (58) des zweiten Dichtelements (16) verbunden ist, so dass in der ersten Dichtstellung ein Wellenberg (48) einer durch den ersten Rand (58) des ersten Dichtelements (16) definierten ersten Wellenlinie (44) in ein Wellental (50) einer durch den ersten Rand (58) des zweiten Dichtelements (16) definierten Wellenlinie (44) eingreift, dadurch gekennzeichnet, dass die erste Wand (42) im Bereich zwischen dem ersten und dem zweiten Rand (58, 60) ein mindestens abschnittsweise umlaufendes Stützelement (62) trägt.
21. Chirurgische Dichtung nach Satz 20, dadurch gekennzeichnet, dass das erste und/oder das zweite Dichtelement (16) in Form eines Dichtelements (16) nach einem der Sätze 2 bis 19 ausgebildet ist.
22. Chirurgisches Abdichtungssystem zum Einführen chirurgischer Instrumente in einen menschlichen oder tierischen Körper, vorzugsweise längs einer vom Abdichtungssystem definierten Längsachse (20), umfassend einen eine Einführöffnung aufweisenden Trokar und ein die Einführöffnung mindestens teilweise verschließendes, eine Öffnung (54) aufweisendes chirurgisches Dichtelement (16) zum Abdichten der Einführöffnung beim Einführen eines Instruments in die Öffnung (54), dadurch gekennzeichnet, dass das Dichtelement (16) ein Dichtelement (16) nach einem der Sätze 1 bis 19 oder eine chirurgische Dichtung nach einem der Sätze 20 oder 21 ist.
23. Chirurgisches Abdichtungssystem nach Satz 22, gekennzeichnet durch eine Dichtelementhalterung (18), welche einerseits mit dem Trokar und andererseits mit dem chirurgischen Dichtelement (16) oder der Dichtung kraft- und/oder formschlüssig verbindbar ist.
24. Chirurgisches Abdichtungssystem nach Satz 22 oder 23, gekennzeichnet durch eine Schutzvorrichtung (146) für das Dichtelement (16) oder die Dichtung.
25. Chirurgisches Abdichtungssystem nach Satz 24, dadurch gekennzeichnet, dass die Schutzvorrichtung (146) einen am Trokar oder an einem Teil desselben anordenbaren, ringförmigen oder im Wesentlichen ringförmigen und eine Durchbrechung definierenden Grundkörper (148) mit mehreren in Umfangsrichtung angeordneten und parallel oder auf eine Längsachse (20) der Schutzvorrichtung (146) hin weisenden Schutzelementen (158, 160) umfasst, welche Schutzelemente (158, 160) im Wesentlichen in distaler Richtung weisende freie Enden (162, 164) aufweisen.
26. Chirurgisches Abdichtungssystem nach Satz 25, gekennzeichnet durch eine am Grundkörper (148) angeordnete Verbindungseinrichtung zum Verbinden der Schutzvorrichtung (146) mit dem chirurgischen Dichtelement (16) oder dem chirurgischen Abdichtungssystem.
27. Chirurgisches Abdichtungssystem nach Satz 25 oder 26, dadurch gekennzeichnet, dass die Schutzelemente (158, 160) mindestens abschnittsweise in einer Grundstellung, in welcher die Öffnung (54) einen minimalen Durchmesser aufweist, am Stützelement (62) anliegen.
28. Chirurgisches Abdichtungssystem nach einem der Sätze 25 bis 27, dadurch gekennzeichnet, dass mindestens 60% der Länge der Schutzelemente (158, 160) sich distalseitig des Stützelements (62) erstreckt, vorzugsweise mindestens 50%.
29. Chirurgisches Abdichtungssystem nach einem der Sätze 25 bis 28, dadurch gekennzeichnet, dass in der Grundstellung die Schutzelemente (158, 160) durch das Stützelement (62) in Richtung auf die Längsachse (20) hin vorgespannt sind.
30. Chirurgisches Abdichtungssystem nach einem der Sätze 25 bis 29, dadurch gekennzeichnet, dass die Schutzelemente (158, 160) in sich flexibel ausgebildet sind.
31. Chirurgisches Abdichtungssystem nach einem der Sätze 22 bis 30, gekennzeichnet durch mindestens einen in Richtung auf die Längsachse (20) oder im Wesentlichen auf die Längsachse hin wirkenden Anschlag (172), welcher das Stützelement (62) in Umfangsrichtung mindestens abschnittsweise umgibt und in der Grundstellung von der das Stützelement (62) von der Längsachse (20) weg weisend begrenzenden Wand (42) beabstandet ist.
32. Chirurgisches Abdichtungssystem nach Satz 31, dadurch gekennzeichnet, dass der mindestens eine Anschlag (172) an einer Dichtelementhalterung (18) und/oder an einem das Dichtelement (16) oder die chirurgische Dichtung umgebenden Gehäuse angeordnet oder ausgebildet ist.

Die nachfolgende Beschreibung bevorzugter Ausführungsformen der Erfindung dient im Zusammenhang mit den Zeichnungen der näheren Erläuterung. Es zeigen:
- Figur 1:: Eine perspektivische schematische Gesamtansicht einer Dichtelementhalterung eines Trokars mit montiertem Dichtelement und montierter Schutzvorrichtung;
- Figur 2:: eine perspektivische Ansicht der Anordnung aus Figur 1 mit teilweise entfernter Abdeckung;
- Figur 3:: eine Explosionsdarstellung der in Figur 1 dargestellten Anordnung;
- Figur 4:: eine Draufsicht auf das Dichtelement der Anordnung aus Figur 1; und
- Figur 5:: eine schematische Längsschnittansicht des in Figur 4 dargestellten Dichtelements mit eingesetzter Schutzvorrichtung beim Abdichten eines Instrumentenschafts.

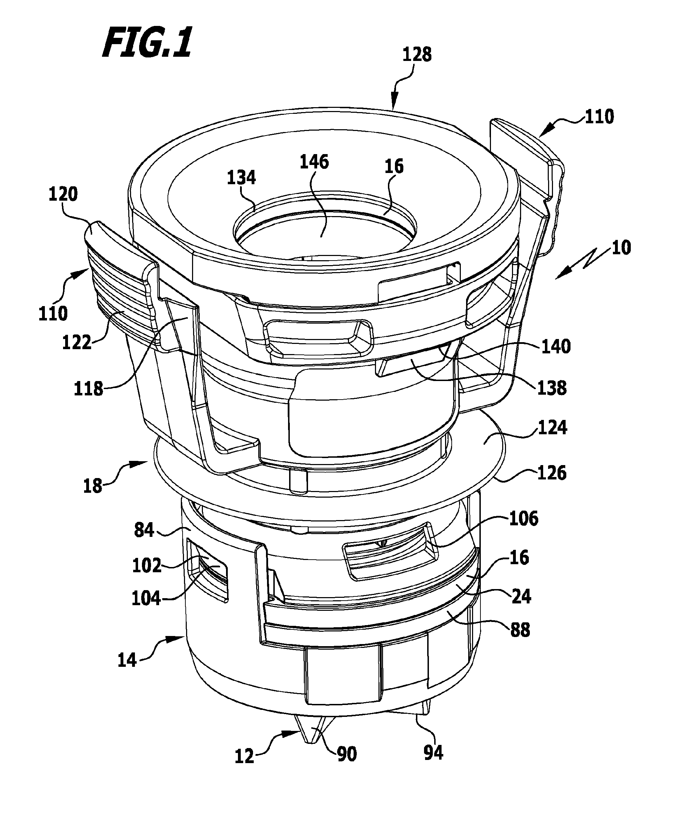

In den Figuren 1 bis 3 ist eine insgesamt mit dem Bezugszeichen 10 bezeichnete chirurgische Dichtungsanordnung schematisch dargestellt. Sie umfasst ein Kreuzschlitzventil 12, welches an einem Haltering 14 gehalten ist, sowie ein Dichtelement 16, welches in seinem prinzipiellen Aufbau im deutschen Gebrauchsmuster 20 2006 005 442 detailliert beschrieben ist. Die dortige Beschreibung wird hiermit in vollem Umfang in die vorliegende Beschreibung mit einbezogen.

Das Dichtelement 16 ist im Inneren einer Dichtelementhalterung 18 gehalten, welche mit dem Haltering 14 lösbar verbindbar ist. Die Dichtelementhalterung 18 ist in ihrem prinzipiellen Aufbau bereits in der EP 2 143 393 A1 detailliert beschrieben. Die dortige Beschreibung wird hiermit in vollem Umfang in die vorliegende Beschreibung mit einbezogen. In der Offenlegungsschrift ist ferner auch beschrieben, wie die Dichtelementhalterung 18 in eine Gehäuse einer Trokarhülse eines Trokars eingesetzt werden kann, so dass hiermit auch diesbezüglich auf die EP 2 143 393 A1 verwiesen wird.

Das Dichtelement 16 ist bezogen auf eine Längsachse 20 der Dichtungsanordnung 10 im Wesentlichen rotationssymmetrisch ausgebildet. Ferner ist es bezogen auf eine quer zur Längsachse 20 verlaufende Öffnungsebene 22 im Wesentlichen spiegelsymmetrisch ausgebildet. Die Öffnungsebene 22 verläuft parallel zu zwei Flanschringen 24, welche das Dichtelement 16 distal- und proximalseitig begrenzen und einen maximalen Außendurchmesser des Dichtelements 16 definieren. Von den Flanschringen 24 stehen maximal weit außen an ihnen und jeweils in die Richtung des anderen Flanschrings 24 hin weisend Ringvorsprünge 26 ab, welche an der Dichtelementhalterung 18 jeweils in distaler und proximaler Richtung abstehende Ringvorsprünge 28 außen umgreifen können. Das Dichtelement 16 kann so auf einfache Weise über die Ringvorsprünge 28 gehängt oder gespannt und im Inneren der Dichtelementhalterung 18 gehalten werden.

Von den Flanschringen 24 erstreckt sich in radialer Richtung auf die Längsachse 20 hin weisend ein erster Querabschnitt 30, welcher in einen ersten, nach außen zurückgebogenen ersten Wulstabschnitt 32 übergeht, welcher wiederum direkt in einen zweiten Wulstabschnitt 34 übergeht, welcher wiederum ein auf die Längsachse 20 hin gerichtetes freies Ende aufweist. Der zweite Wulstabschnitt 34 definiert so eine in Richtung auf die Längsachse 20 hin geöffnete Ringnut 36.

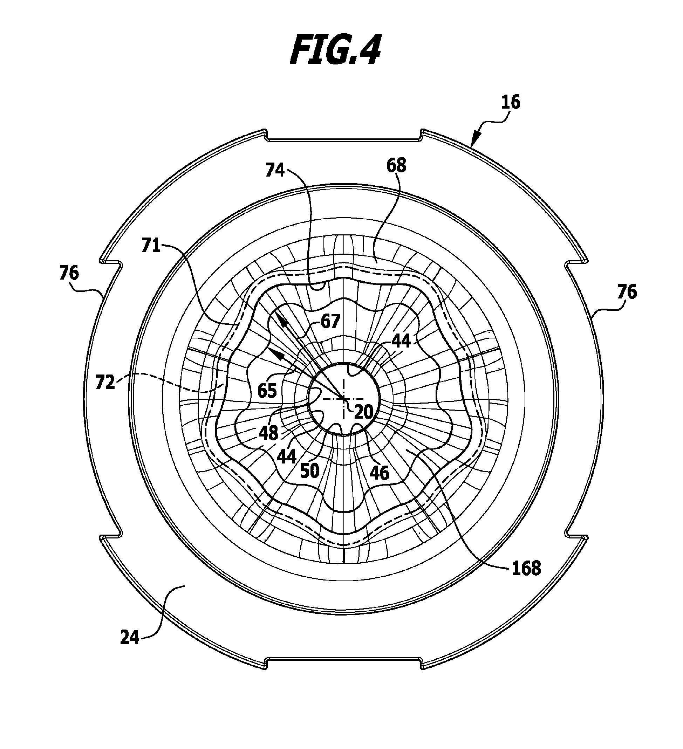

Distalseitig schließt sich an den zweiten Wulstabschnitt 34 ein kurzer zylindrischer Abschnitt 38 an, welcher in einen verdickten, außen am Dichtelement 16 abstehenden, massiven Wulst 40 übergeht. Ausgehend von den Wulsten 40, von denen eine Wand 42 des Dichtelements 16 im Wesentlichen faltenfrei absteht, faltet sich die Wand 42 gardinenartig, also knickfrei, bis zur Öffnungsebene 22 hin. Durch die Faltung entsteht eine Dichtlinie 46, welche in Form einer Wellenlinie 44 Wellenberge 48 proximalseitig der Öffnungsebene 22 und Wellentäler 50 distalseitig der Öffnungsebene 22 definiert. Die Wellenlinie 44 ist etwas verstärkt und in Form einer Dichtlippe 52 ausgebildet, welche sich somit teilweise proximalseitig und teilweise distalseitig der Öffnungsebene 22 befindet. In einer Draufsicht, wie sie schematisch in Figur 4 dargestellt ist, ist jedoch erkennbar, dass die Dichtlinie 46 und damit auch die Dichtlippe 52 eine kreisförmige Öffnung 54 des Dichtelements 16 begrenzen. Die Öffnung 54 weist in einer Grundstellung, wie sie beispielsweise in Figur 4 dargestellt ist, einen minimalen Innendurchmesser auf. Die Öffnung 54 kann aufgrund ihrer Konstruktion so weit aufgeweitet werden, dass ein Innendurchmesser derselben einem Innendurchmesser des Dichtelements 16 im Bereich der Wulste 40 entspricht. Die gefaltete Wand 42 entfaltet sich dabei, ist praktisch auf der gesamten Länge zwischen den Wulsten 40 vollständig entfaltet und definiert so eine im Wesentlichen zylindrische Wandfläche.

Zur Stabilisierung des Dichtelements 26 sind auf einer Außenseite der Wand 42 ausgehend von den Wulsten 40 bis an die Dichtlippe 52 heranreichende Verstärkungsrippen 56 ausgebildet.

Ferner ist zwischen einem die Öffnung 54 begrenzenden ersten Rand 58, welcher im Wesentlichen durch die Dichtlippe 52 definiert wird, und einem vom Flanschring 24 definierten zweiten Rand 60 ein um die Längsachse 20 umlaufendes Stützelement 62 ausgebildet, und zwar in Form eines in Richtung auf die Längsachse 20 hin weisenden Vorsprungs 64, welcher von einer Innenseite 66 der Wand 42 absteht. Das Stützelement weist eine im Wesentlichen konstante Dicke auf, so dass es, analog der Innenseite 66, einen sich in Abhängigkeit einer Winkelstellung ändernden Abstand von der Längsachse aufweist. Exemplarisch für die so definierte Abstandfunktion sind in Figur 4 schematisch ein minimaler Abstand 65 und ein maximaler Abstand 67 des Stützelements 62 von der Längsachse eingezeichnet. Diese beiden genannten Abstände 65 und 67 definieren dann ein Minimum beziehungsweise ein Maximum der Abstandsfunktion des Abstands in Abhängigkeit des Umfangswinkels. Aufgrund der besonderen Faltung der Wand 42 weist die Abstandsfunktion des in Figur 4 dargestellten Ausführungsbeispiels 10 Minima und 10 Maxima auf.

Der Vorsprung 64 definiert ferner einen im Vergleich zur Wand 42 in diesem Bereich etwas abgeflachten Innenflächenbereich 68, welcher im Wesentlichen in proximaler Richtung weist und sich distalseitig des Wulsts 40 einen Teil der Innenseite 66 bildet. Der Innenflächenbereich 68 geht in einen sich mehr parallel zur Längsachse 20 erstreckenden Abschnitt 70 über, welcher mit einer den Wulst 40 distalseitig begrenzenden und in distaler Richtung weisenden Ringfläche 72 eine Ringkante 74 definiert, welche abgerundet ist. So ergibt sich insgesamt eine in Richtung auf die Längsachse hin konvex gewölbte Stützfläche 71, die durch den Innenflächenbereich 68 und den Abschnitt 70 definiert wird. Die Funktion und Wirkungsweise des Stützelements 62 wird später noch im Einzelnen erläutert.

Das Dichtelement 16 ist insgesamt einstückig aus einem Kunststoff gespritzt, welcher eine ausreichende Flexibilität aufweist, um eine Auffaltung der Wand 42 in der beschriebenen Weise zu ermöglichen. Vorzugsweise kann der Kunststoff auch elastomerische Eigenschaften aufweisen.

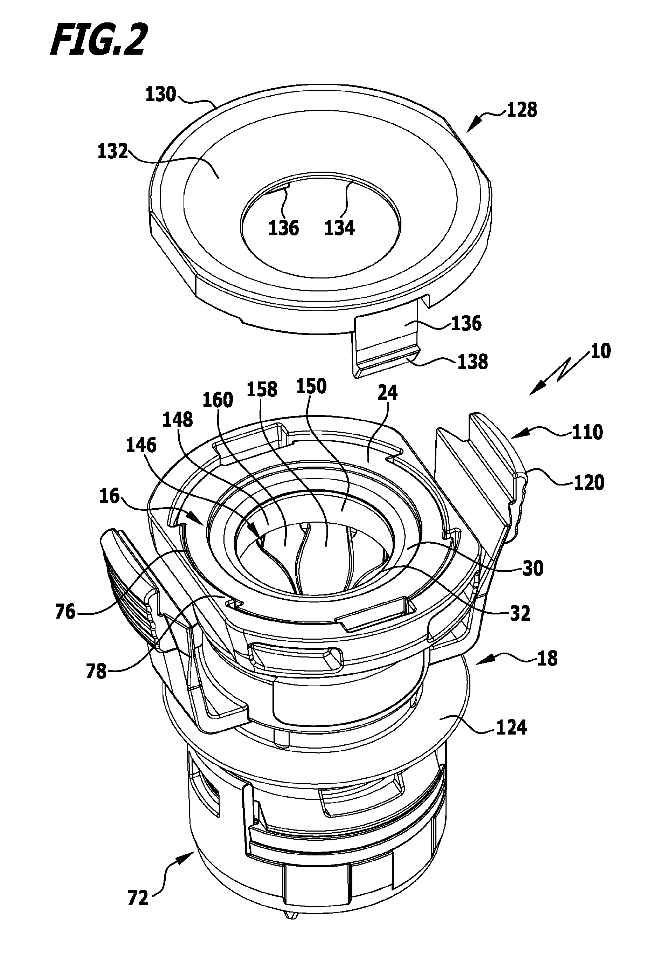

Ferner sind an den Flanschringen 24 jeweils zwei, einander diametral gegenüberliegende Aussparungen 76 vorgesehen, welche korrespondierend zu zwei in radialer Richtung von der Dichtelementhalterung 18 in Richtung auf die Längsachse 20 hin vorstehenden Vorsprüngen 78 ausgebildet sind. Die Aussparungen 76 in Verbindung mit den Vorsprüngen 78 bilden eine Verdrehsicherung, so dass das Dichtelement 16 und die Dichtelementhalterung 18 in einer beispielsweise in den Figuren 1 und 2 dargestellten Montagestellung nicht um die Längsachse 22 relativ zueinander verdrehbar sind.

Der Haltering 14 umfasst einen im Querschnitt kreisförmigen Ring 80, von dessen proximalseitigem Rand ein in proximaler Richtung abstehender ringförmiger Flansch 82 ausgebildet ist, welcher sich jedoch nicht über eine gesamte Breite einer Wand des Rings 80 erstreckt, sondern nur etwa über die Hälfte. Vom proximalen Rand des Rings 80 stehen ferner zwei zueinander symmetrisch ausgebildete Verbindungsflügel 84 einander, bezogen auf die Längsachse 20, diametral gegenüberliegend in proximaler Richtung ab. Die Verbindungsflügel 84 weisen jeweils zwei im Wesentlichen rechteckige Durchbrechungen 86 auf, welche quer zur Längsachse 22 orientiert sind. Die Verbindungsflügel 84 sind vom Flansch 82 etwas beabstandet angeordnet, so dass zwischen dem Flansch 82 und den Verbindungsflügeln 84 jeweils eine Nut ausgebildet ist.

Das Kreuzschlitzventil 12 umfasst proximalseitig einen Befestigungsflansch 88, welcher einen in distaler Richtung weisenden Ringvorsprung 94 trägt, welcher in seiner Höhe sowie in seinen äußeren Abmessungen korrespondierend zu den Nuten am Haltering 14 ausgebildet ist. Das Kreuzschlitzventil 12 umfasst ferner einen am Befestigungsflansch 88 in distaler Richtung abstehenden Ventilkörper 90, welcher distalseitig in eine kreuzförmige Endfläche mündet, die mit zwei zueinander senkrechten Schlitzen versehen ist. Der Ventilkörper 90 ist in einer Grundstellung so ausgebildet, dass durch Schlitze getrennte Schnittflächen des Ventilkörpers 90 direkt aneinander anliegen und so eine durch den Befestigungsflansch 88 definierte ringförmige Öffnung etwas distalseitig des Befestigungsflanschs 88 vollständig verschließen. Der Ventilkörper 90 ist proximalseitig direkt an einen Innenrand des Befestigungsflansch 88 angeformt, so dass zwischen dem Ventilkörper 90 und dem Ringvorsprung des Befestigungsflanschs eine Ringnut ausgebildet ist, in welche der Flansch 82 im Wesentlichen formschlüssig eingreifen kann.

Der Befestigungsflansch 88 ist ferner mit zwei in radialer Richtung weisenden Aussparungen 92 versehen, in welche die Verbindungsflügel 84 eingreifen, wenn der Ventilkörper 90 in den Haltering 14 eingesetzt ist und mindestens teilweise mit dem Ventilkörper 90, insbesondere mit dessen die Schlitze aufweisender Endfläche 94, über einen distalseitigen Rand des Halterings 14 vorsteht.

Die Dichtelementhalterung 18 ist im Wesentlichen langgestreckt hülsenförmig ausgebildet. Sie umfasst einen zentralen, koaxial zur Längsachse 20 ausgebildeten Hülsenkörper 96. Eine Innenfläche 98 des Hülsenkörpers 96 ist vollständig rotationssymmetrisch ausgebildet. Die Innenfläche 98 definiert und begrenzt einen Längskanal 100, in welchen das Dichtelement 16 eingesetzt ist. Ein Innendurchmesser des Hülsenkörpers 96 erweitert sich jeweils zum distalen und proximalen Ende desselben hin etwas. Distalseitig und proximalseitig sind jeweils die ringförmigen, in proximaler beziehungsweise distaler Richtung weisenden Ringvorsprünge 28 ausgebildet zum in Eingriff Bringen mit den Flanschringen 24 des Dichtelements 16.

Auf einer Außenseite des Hülsenkörpers 96 sind etwas proximalseitig des distalseitigen Ringvorsprungs 28 zwei, bezogen auf die Längsachse 20 einander diametral gegenüberliegende und in entgegengesetzte Richtungen weisende Rastnasen 102 ausgebildet, welche nach außen weisende, etwas in distaler Richtung geneigte Aufgleitflächen 104 und somit auch eine in proximaler Richtung weisende Ringkante definieren. Die Rastnasen 102 sind korrespondierend zu den Durchbrechungen 86 an den Verbindungsflügeln 84 ausgebildet. Die Verbindungsflügel 84 können von distal her kommend über die Aufgleitflächen 104 geschoben werden, so dass sie etwas in radialer Richtung von der Längsachse 20 weg nach außen ausschwenken. Sobald die Rastnasen 102 ganz in die Durchbrechungen 86 eingreifen können, federn die Verbindungsflügel 84 wieder in Richtung auf die Längsachse 20 zurück. Auf die beschriebene Weise können der Haltering 14 und die Dichtelementhalterung 18 rastend miteinander verbunden werden. Die Verbindung erfolgt jedoch erst dann, wenn die Dichtelementhalterung 18 mit dem Dichtelement 16 und der Halterring 14 mit dem Kreuzschlitzventil 12 bestückt sind, so dass der Befestigungsflansch 88 und der Flanschring 24 direkt aneinander anliegen.

Zwischen den Rastnasen 102, also um 90° relativ zu diesen in Umfangsrichtung versetzt, sind am Hülsenkörper 96 zwei rechteckige Durchbrechungen 106 vorgesehen, welche einen Innenraum 108 des Hülsenkörpers 96 mit einer Außenseite desselben verbinden. Auf diese Weise kann ein Druckausgleich zwischen dem Innenraum 108 und einer Umgebung der Dichtelementhalterung 18 erreicht werden. Der so erreichbare Druckausgleich zwischen einem im Körper eines Patienten und im Innenraum 108 herrschenden Gasdrucks beziehungsweise die so mögliche End-/Belüftung des Innenraums 108 verhindert, dass eine Aufweitung des Dichtelements 16 gegen ein Gasvolumen im Innenraum erfolgen muss, welches nach Montage des Dichtelements 16 an der Dichtelementhalterung 18 eingeschlossen würde.

Zum Verbinden der Dichtelementhalterung 18 mit einem in den Figuren nicht dargestellten Dichtungsgehäuse einer ebenfalls nicht dargestellten Trokarhülse sind von einer Außenseite der Dichtelementhalterung 18 zwei Kupplungsglieder 110 einander diametral gegenüberliegend abstehend angeordnet. Sie umfassen jeweils einen direkt vom Hülsenkörper 96 in radialer Richtung abstehenden Quersteg 112, von welchem sich ein im Wesentlichen parallel zum Hülsenkörper 96 in proximaler Richtung erstreckender Federteil 114 weg erstreckt. An einem proximalen Ende des Federteils 114 sind beidseits des Federteils 114 im Wesentlichen in Umfangsrichtung weisende, seitlich überstehende Rastvorsprünge 116 ausgebildet, welche jeweils von der Längsachse 20 weg weisende Aufgleitflächen 118 definieren. Zwischen den Aufgleitflächen 118 ist auf einer Außenseite der Federteile 114 ein im Wesentlichen quaderförmiges Bedienelement 120 angeordnet, welches proximalseitig etwas über das Ende des Federteils 114 vorsteht. Auf einer Außenseite ist das Bedienelement 120 mit einer Oberflächenstruktur 122 versehen, welche mehrere parallel zueinander und in Umfangsrichtung verlaufende Nuten umfasst, die die Gefahr eines Abrutschen eines Fingers einer Bedienperson beim Bewegen der Bedienelemente 120 in Richtung auf die Längsachse 20 hin deutlich verringern.

Zum Verbinden der Dichtelementhalterung 18 mit dem nicht dargestellten Dichtungsgehäuse wird das distale Ende der Dichtelementhalterung 18 in das Dichtungsgehäuse eingeführt. Dabei gleiten die Federteile 114 an Vorsprüngen des Dichtungsgehäuses auf und schwenken etwas in Richtung auf die Längsachse 20 hin. Sobald die Dichtelementhalterung 18 ihre axiale Position im Dichtungsgehäuse einnimmt, können die Federteile 114 in am Dichtungsgehäuse vorgesehene Hinterschneidungen eintauchen, so dass die Federteile 114 in radialer Richtung etwas nach außen ausfedern können. Zum Lösen der Dichtelementhalterung 18 von der Trokarhülse können die Bedienelement 120 mit einer in Richtung auf die Längsachse 20 gerichteten Kraft beaufschlagt werden, so dass die Federteile 114 in Richtung auf die Längsachse 20 verschwenkt werden und die Rastvorsprünge 116 die Hinterschneidungen am Dichtungsgehäuse freigeben. Die Dichtelementhalterung 18 kann dann in proximaler Richtung aus dem Dichtungsgehäuse herausgezogen werden.

Etwas distalseitig der Querstege 112 ist an der Dichtelementhalterung 18 ferner ein Halterungsdichtelement 124 ausgebildet, und zwar in Form eines im Wesentlichen in radialer Richtung abstehenden Ringflansches, welcher etwas in distaler Richtung geneigt ist, und zwar um etwa 2° bezogen auf eine senkrecht zur Längsachse 20 verlaufende Querebene. Das Halterungsdichtelement 124 weist eine Dicke auf, welche noch eine gewisse Elastizität beziehungsweise Flexibilität des Halterungsdichtelements 124 vorgibt. Es kann so in axialer Richtung federn und Fertigungstoleranzen an der Trokarhülse und der Dichtelementhalterung 18 gut ausgleichen. Das Halterungsdichtelement 124 ist an der Dichtelementhalterung 18 derart angeordnet, dass dann, wenn die Dichtelementhalterung 18 rastend in der beschriebenen Weise mit dem Dichtungsgehäuse verbunden ist, eine in distaler Richtung weisende Dichtfläche 126 der Dichtelementhalterung 18 an einer in proximaler Richtung weisenden Ringfläche des Dichtungsgehäuses der Trokarhülse, optional etwas vorgespannt, anliegt und so eine perfekte Abdichtung der Dichtelementhalterung 18 bezogen auf eine Innenwand des Dichtungsgehäuses der Trokarhülse erreicht wird. Das Halterungsdichtelement 124 kann zur Verbesserung einer Abdichtwirkung optional eine weitere, in den Figuren nicht dargestellte Dichtung tragen, welche beispielsweise durch Anspritzen eines Elastomers hergestellt werden kann, und zwar auf oder an der Dichtfläche 126.

Zum Verschließen des nicht dargestellten Dichtungsgehäuses dient ein insgesamt mit dem Bezugszeichen 128 versehener Deckel. Er umfasst einen ringförmigen Rahmen 130, von dem sich im Inneren und in distaler Richtung eine sich im Durchmesser konisch verjüngende Deckelfläche 132 bis zu einer Deckelöffnung 134 erstreckt, welche einen maximalen Innendurchmesser der Dichtungsanordnung 10 definiert. Instrumente mit Schaftdurchmessern, welche größer sind als ein Innendurchmesser der Deckelöffnung 134, können folglich nicht in die Trokarhülse eingeführt werden.

Der Deckel 128 weist ferner zwei in distaler Richtung weisende, einander gegenüberliegende Laschen 136 auf, welche an freien Enden Rastvorsprünge 138 aufweisen, welche mit korrespondierenden Rastkanten 140 an der Dichtelementhalterung 18 in Eingriff gebracht werden können. Der Deckel 128 kann dann nach Montage der Dichtelementhalterung 18 am Dichtungsgehäuse auf einfache Weise auf die Dichtelementhalterung 18 aufgeschnappt werden.

Im proximalseitigen Wulst 40 sind fünf gleichmäßig über den Umfang verteilte, in radialer Richtung auf die Längsachse 20 hin geöffnete und Verbindungsglieder bildende Vertiefungen 142 vorgesehen, welche der Aufnahme korrespondierender Verbindungselemente 144 einer insgesamt mit dem Bezugszeichen 146 bezeichneten Schutzvorrichtung dienen. Die zum Schutz des Dichtelements 16 vorgesehene Schutzvorrichtung 146 umfasst einen ringförmigen, in sich geschlossenen Grundkörper 148, welcher eine kreisförmige Durchbrechung 150 definiert. Vom Grundkörper 148 steht ein in radialer Richtung nach außen weisend und benachbart einem proximalen Ende 152 der Schutzvorrichtung 146 ein Ringvorsprung 154 ab. Etwas weiter distalseitig sind die Verbindungselemente 144 in Form kurzer stegartiger Vorsprünge angeordnet. Sie erstrecken sich im Umfang über etwa ein Zehntel des Gesamtumfangs des Grundkörpers 148 und sind korrespondierend zu den Vertiefungen 142 ausgebildet. Der Grundkörper 148 kann somit direkt am Dichtelement 16 gelagert werden, wobei hierfür die Verbindungselemente 144 formschlüssig in die Vertiefungen 142 eingreifen. Sie bilden somit gleichzeitig eine Verdrehsicherung der Schutzvorrichtung 146 relativ zum Dichtelement 16.

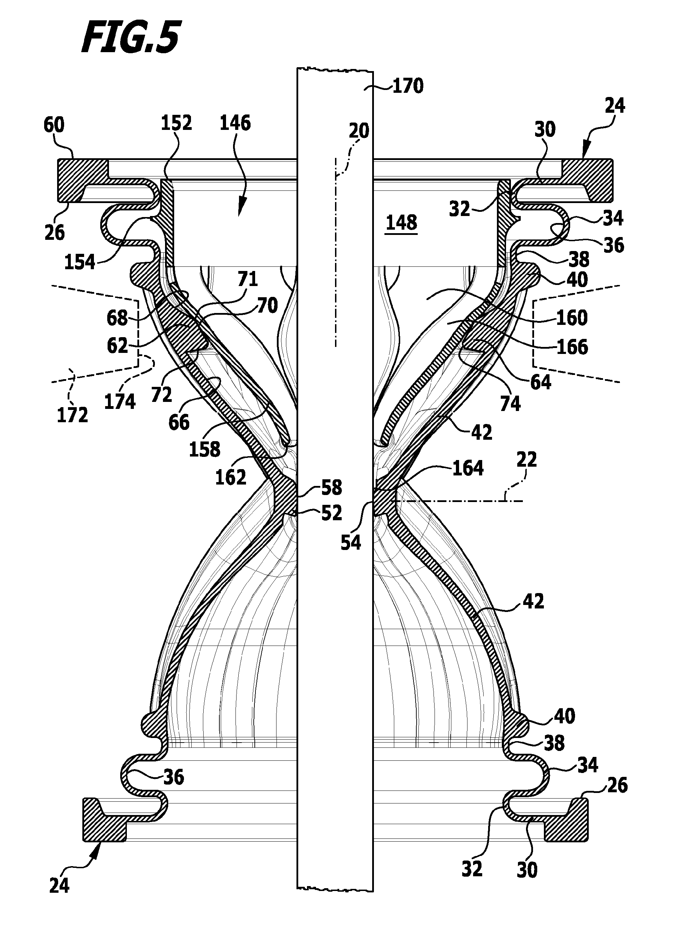

Von einem distalseitigen Rand 156 des Grundkörpers 148 erstrecken sich insgesamt zehn, und zwar jeweils fünf lamellenförmige kurze Schutzelemente 158 und fünf lange Schutzelemente 160 in distaler Richtung. Sie weisen im Längsschnitt eine über ihre gesamte Länge konstante Dicke auf, wie beispielsweise schematisch in Figur 5 dargestellt. Die kurzen Schutzelemente 158 sind im Wesentlichen bis zu ihrem freien Ende 162 nahezu gleich breit, die langen Schutzelemente 160 in etwa auf derselben Länge wie die kurzen Schutzelemente 158, allerdings nimmt dann eine Breite der langen Schutzelemente 160 auf deren distales Ende 164 hin deutlich ab, so dass ein schmaler Schutzelementabschnitt 166 ausgebildet wird, welcher in seiner Außenkontur im Wesentlichen korrespondierend zu einem Wellental 50 ausgebildet ist.

Die langen Schutzelemente 160 können optional jeweils ein von einer Außenseite etwas in distaler Richtung geneigt abstehendes Rückhalteelement aufweisen, welches bevorzugt eine Länge von weniger als 1mm aufweist. Das Rückhalteelement ist im Wesentlichen kegelstumpfförmig ausgebildet und weist eine abgerundete Spitze auf.

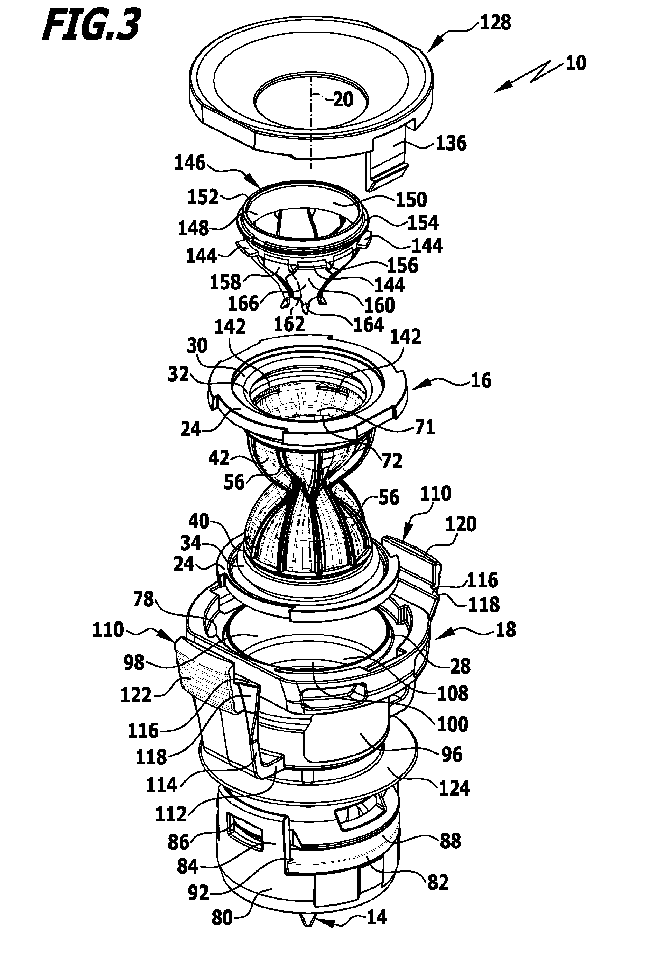

Wenn der Grundkörper 148 in der oben beschriebenen Weise mit dem Dichtelement 16 verbunden ist, falten sich die aufgrund ihrer geringen Dicke flexiblen Schutzelemente 158 und 160 in Richtung auf die Längsachse 20 hin übereinander und nehmen die in Figur 3 dargestellte Stellung ein. Es sei angemerkt, dass sich die langen Schutzelemente 160 vorzugsweise distalseitig der Verbindungselemente 144 vom Rand 156 weg erstrecken, die kurzen Schutzelemente 158 in den Bereichen des Rands 156, zu welchem kein Verbindungselement 144 korrespondiert. Durch die entsprechend vorgesehenen Vertiefungen 142 kann die Schutzvorrichtung 146 positionsrichtig mit dem Dichtelement 16 verbunden werden. Dies bedeutet, dass in einer Grundstellung alle fünf Schutzelementabschnitte 166 in korrespondierende Wellentäler 50 eintauchen. Damit ist sichergestellt, dass die distalen Enden 162 und 164 der Schutzvorrichtungen 146 praktisch bis an die Dichtlinie 46 heranreichen und im Wesentlichen eine innere Wandfläche 168 des Dichtelements 116 vollständig verdecken.

Die Schutzelemente 158 und 160 stehen in der montierten Stellung bereits wenig distalseitig des Wulsts 40 von der Wandfläche 168 ab und liegen im Bereich proximaler Abschnitte derselben am Stützelement 62 an, und zwar, wie in Figur 5 schematisch dargestellt, im Wesentlichen im Bereich im Übergang zwischen dem Innenflächenbereich 68 und dem Abschnitt 70, also an der Stützfläche 71. Das Stützelement 62 ist so angeordnet und bemessen, dass die Schutzelemente 158 und 160 etwas in Richtung auf die Längsachse 20 hin vorgespannt werden und sich mit vorzugsweise mindestens 60% ihrer Länge, bevorzugt maximal 80%, distalseitig des Stützelements 62 erstrecken.

Im Vergleich zur unmontierten Stellung, wie sie schematisch in Figur 3 dargestellt ist, sind die Enden 162 und 164 etwas von der Wandfläche 168 beabstandet. Wird ein Instrument mit einem langgestreckten Schaft 170, wie schematisch in Figur 5 dargestellt, von proximal her kommend, in die Dichtungsanordnung 10 durch die Deckelöffnung 134 eingeführt, so tritt der Schaft 170 zunächst in Kontakt mit Innenflächen der kurzen Schutzelemente 158. Ist ein Außendurchmesser eines Instruments größer als die Öffnung 54, dann werden die kurzen Schutzelemente 158 gegen die langen Schutzelemente 160 gedrückt und mit diesen nach außen geschwenkt. Das Stützelement 62 dient dabei als Widerlager, wie schematisch in Figur 5 dargestellt. Durch von den Schutzelementen 158 und 160 auf das Stützelement 62 übertragene Kräfte kann dieses im Vergleich zu einer Grundstellung gedehnt werden, und zwar maximal um 25%, vorzugsweise in einem Bereich von 10% bis 20%. Ein freier Innendurchmesser, der im Wesentlichen definiert wird durch einen Durchmesser der Ringkante 74, kann dann folglich in den angegebenen Aufweitungsbereichen aufgedehnt werden.

Das Stützelement 62 verbessert zudem eine Rückstellung der Schutzelemente 158 und 160 der Schutzvorrichtung 146 von einer ausgelenkten Stellung zurück in die Grundstellung und ermöglicht zudem eine aktive Unterstützung einer seitlichen Auslenkung des Dichtelements 16. Eine Rückstellungsfunktion für die lamellenförmigen Schutzelemente 158 und 160 wird somit direkt durch das Dichtelement 16, und zwar dessen Stützelement 62, übernommen oder zumindest unterstützt. Durch das beispielsweise in Form eines Verstärkungsrings angespritzte Stützelement 62 wird eine leichte Vorspannung in Richtung auf die Längsachse 20 hin auf die Schutzelemente 158 und 160 ausgeübt.

Die Schutzvorrichtung 146, insbesondere die Schutzelemente 158 und 160, ist vorzugsweise aus einem Kunststoff hergestellt, bevorzugt aus LDPE, Polypropylen oder einem thermoplastischen Polymer. Das Stützelement 62 weist zumindest teilweise einen Elastomeranteil auf, so dass eine elastische Verformung desselben genutzt werden kann, um die Schutzelemente 158 und 160 nach Entnahme des Instruments wieder in ihre ursprüngliche Form zu bringen.

Des Weiteren unterstützt das Stützelement 62 in Form eines Widerlagers eine Verkippbewegung der Schutzvorrichtung 146 in Richtung einer Auslenkung des Schafts 170. Durch die beschriebene definierte Verbindung zwischen der Schutzvorrichtung 146 und dem Dichtelement 16 wird infolge einer Auslenkung der Schutzvorrichtung 146 das Dichtelement 16 dem Instrument, insbesondere dessen Schaft 170, nachgeführt. Ferner kann das Stützelement 62 auch eine Wider- oder Gegenlagerfunktion für ein Verkippen des Dichtelements 62 insgesamt übernehmen, und zwar insbesondere im Zusammenwirken mit einem schematisch in Figur 5 gestrichelt gezeichneten Anschlag 172, welcher eine im Wesentlichen in Richtung auf die Längsachse 20 hin weisende Anschlagfläche 174 aufweist. Optional kann die Anschlagfläche 174 auch in mehrere Anschlagflächenbereiche unterteilt sein, beispielsweise dadurch, dass nicht nur ein in Umfangsrichtung das Stützelement 62 umgebender Anschlag 172 vorgesehen ist, sondern mehrere Anschläge mit voneinander getrennten Anschlagflächen. Der Anschlag 172 kann beispielsweise an der Trokarhülse oder an der Dichtelementhalterung 18 angeordnet oder ausgebildet sein. Denkbar wäre es auch, den Anschlag 172 an einer Gehäuseinnenwand eines die Dichtelementhalterung 18 umgebenden Gehäuses anzuordnen. Es kann sich bei der Anschlagfläche 174 um eine Ringfläche oder einen Teil der Innenfläche 98 handeln, welche auf die Längsachse oder im Wesentlichen auf die Längsachse der Trokarhülse oder der Dichtelementhalterung 18 hin weist. Wird infolge einer seitlichen Auslenkung des Schafts 170 eines eingeführten Instruments das Dichtelement 16 mit der Wand 42 im Bereich des Stützelements 62 gegen die Anschlagfläche 172 beziehungsweise die Anschlagflächen gedrückt, bewirkt eine weitere Auslenkung eine Verkippung des Dichtelements 62 insgesamt längs der Längsachse um einen Drehpunkt, welcher im Wesentlichen durch den Kontakt zwischen Anschlagfläche 172 und der ersten Wand 42 im Bereich des Stützelements 62 definiert wird. Dieses Prinzip ist besonders vorteilhaft für ein Dichtelement 16 mit einer knickfrei gefalteten oder gewellten Wand 42, welche bedingt durch ihre Faltung nur eine geringe Umfangsspannung im Dichtbereich, das heißt im Bereich der Dichtlinie 46 beziehungsweise der Dichtlippe 52, aufweist. Dadurch kann durch Mitwirkung des Stützelements 62 eine Undichtigkeit zwischen dem Schaft 170 und der Öffnung 54 bei seitlicher Auslenkung des Schafts 170 im Wesentlichen verhindert werden.

Ein weiterer Vorteil des Stützelements 62 besteht darin, dass eine bessere Zentrierung des Schafts 170 beim Einführen erreicht werden kann. Durch diese Zentrierwirkung wird wiederum auch die Gefahr eines versehentlichen Einstechens des Schafts 170 in das Dichtelement 16 deutliche reduziert.

Des Weiteren werden bei einer Verkippung beziehungsweise Auslenkung der langen Schutzelemente 160 im Bereich von deren Schutzelementabschnitt 166 angeordnete Rückhalteelemente mit ihren Spitzen in die Wand 42 des Dichtelements 16 eingedrückt. Dies führt partiell zu einer Auswölbung der Wand 42 durch die Rückhalteelemente, so dass sich diese in der Wand 42 quasi verhaken, man kann auch sagen, die Rückhalteelemente und das Dichtelement 16 stehen miteinander in Eingriff. Durch das Verhaken der Rückhalteelemente in der Wand 42 wird ferner eine Relativbewegung zwischen den distalen Enden 164 der langen Schutzelemente 160 und dem Dichtelement 16 praktisch verhindert. Unabhängig von einer Aufweitung oder Auffaltung der Wand 42 in Abhängigkeit eines Durchmessers des eingeführten Instruments reichen die distalen Enden 164 der langen Schutzelemente 160 stets bis an die Dichtlinie 46 heran und schützen das Dichtelement 16 vor den eingangs beschriebenen möglichen Beschädigungen infolge des in Kontakt Tretens der Wand 42 mit spitzen Kanten des Schafts 170.

Ferner kann durch die Wölbung der Schutzelemente 158 und 160 schwach konvex von der Wand 42 weg sichergestellt werden, dass ein eingeführtes Instrument zunächst mit distalen Endbereichen der Schutzelemente 158 und 160 in Kontakt tritt, bevor es die Dichtlippe 52 berühren kann.

Das Vorsehen des Stützelements 62 am Dichtelement 16 hat im Vergleich zu anderen, bislang eingesetzten Dichtelementen insbesondere die Vorteile, dass eine Rückstellung der Schutzelemente 158 und 160 der Schutzvorrichtung 146 nach vorangegangener Aufdehnung und nach Entfernen eines Instruments verbessert wird, des Weiteren eine höhere Dichtigkeit bei seitlicher Auslenkung von Instrumenten, insbesondere Instrumenten mit kleinen Schaftdurchmessern, erreicht wird ebenso wie eine Zentrierfunktion der Schutzvorrichtung 146 beim Einführen von Instrumenten verbessert wird. Eine mögliche Beschädigung des Dichtelements 16 durch spitze oder scharfkantige Instrumentenabschnitte ist im Vergleich zu herkömmlichen Dichtelementen deutlich reduziert.

## Patentansprüche

1. Chirurgisches Dichtelement (16), insbesondere für einen Trokar und/oder zum Abdichten von Schäften (170) langgestreckter chirurgischer Instrumente beim Einführen in einen menschlichen oder tierischen Körper, welches eine Längsachse (20) definiert und eine im Durchmesser veränderliche und quer oder im Wesentlichen quer zur Längsachse orientierte Öffnung (54) aufweist, durch die ein Instrument einführbar ist, und eine ringförmig geschlossene, flexible Wand (42) umfasst, welche Wand (42) einen ersten und einen zweiten, jeweils in sich geschlossenen Rand (58, 60) aufweist, wobei der erste Rand (58) die Öffnung (54) begrenzt, **dadurch gekennzeichnet, dass** die Wand (42) im Bereich zwischen dem ersten und dem zweiten Rand (58, 60) ein mindestens abschnittsweise umlaufendes Stützelement (62) trägt.

2. Chirurgisches Dichtelement nach Anspruch 1, **dadurch gekennzeichnet, dass** das Stützelement (62) in Richtung auf die Längsachse (20) vorspringend an der Wand (42) angeordnet oder ausgebildet ist, dass das Stützelement (62) in Form eines Vorsprungs ausgebildet ist und dass das Stützelement (62) auf einer der Längsachse (20) zugewandten Innenseite (66) der Wand (42) angeordnet oder ausgebildet ist.

3. Chirurgisches Dichtelement nach einem der voranstehenden Ansprüche, **dadurch gekennzeichnet, dass** das Stützelement (62) und die Wand (42) einstückig ausgebildet sind.

4. Chirurgisches Dichtelement nach einem der voranstehenden Ansprüche, **dadurch gekennzeichnet, dass** das Stützelement (62) eine mindestens teilweise auf die Längsachse hin weisende Stützfläche (68, 70) für Schutzelemente (158, 160) einer Schutzvorrichtung (146) des Dichtelements (16) aufweist.

5. Chirurgisches Dichtelement nach einem der voranstehenden Ansprüche, **dadurch gekennzeichnet, dass** das Stützelement (62) in Form eines in sich geschlossenen Rings ausgebildet ist.

6. Chirurgisches Dichtelement nach einem der voranstehenden Ansprüche, **dadurch gekennzeichnet, dass** ein Abstand des Stützelements (62) von der Längsachse (20) in einer Grundstellung des Dichtelements (16), in welcher die Öffnung (54) einen minimalen Durchmesser aufweist, in Abhängigkeit eines Umfangswinkels bezogen auf die Längsachse (20) variiert.

7. Chirurgisches Dichtelement nach einem der voranstehenden Ansprüche, **dadurch gekennzeichnet, dass** das Stützelement (62) ausgehend von einer Grundstellung, in welcher die Öffnung (54) einen minimalen Durchmesser aufweist, beim Übergang in eine Einführstellung, in welcher die Öffnung (54) einen maximalen Durchmesser aufweist, maximal um 100% dehnbar ist, vorzugsweise maximal um 10 bis 50%.

8. Chirurgische Dichtung, insbesondere für einen Trokar und/oder zum Abdichten von Schäften (170) langgestreckter chirurgischer Instrumente beim Einführen in einen menschlichen oder tierischen Körper, wobei die Dichtung ein erstes Dichtelement (16) umfasst, welches eine Längsachse (20) definiert, mit einer im Durchmesser veränderlichen und quer oder im Wesentlichen quer zur Längsachse (20) orientierten Öffnung (54) versehen ist, durch die ein Instrument eingeführt werden kann, und eine erste ringförmig geschlossene, flexible Wand (42) umfasst, wobei die erste Wand (42) einen ersten und einen zweiten, jeweils in sich geschlossenen Rand (58, 60) aufweist und wobei der erste Rand (58) die Öffnung (54) begrenzt, wobei die erste Wand (42) in einer ersten Dichtstellung derart wellenförmig ohne Knicke mit in Richtung auf den ersten Rand (58) hin verlaufenden Faltlinien gefaltet ist, dass der erste Rand (58) eine Wellenlinie (44) definiert, welche vollständig auf einer Zylinderfläche liegt, wobei ein zweites, identisch oder im Wesentlichen identisch mit dem ersten Dichtelement (16) ausgebildetes Dichtelement (16) mit einer zweiten Wand (42), die auch einen ersten und zweiten Rand (58, 60) aufweist, vorgesehen ist und wobei der erste Rand (58) des ersten Dichtelements (16) mit dem ersten Rand (58) des zweiten Dichtelements (16) verbunden ist, so dass in der ersten Dichtstellung ein Wellenberg (48) einer durch den ersten Rand (58) des ersten Dichtelements (16) definierten ersten Wellenlinie (44) in ein Wellental (50) einer durch den ersten Rand (58) des zweiten Dichtelements (16) definierten Wellenlinie (44) eingreift, **dadurch gekennzeichnet, dass** die erste Wand (42) im Bereich zwischen dem ersten und dem zweiten Rand (58, 60) ein mindestens abschnittsweise umlaufendes Stützelement (62) trägt.

9. Chirurgische Dichtung nach Anspruch 8, **dadurch gekennzeichnet, dass** das erste und/oder das zweite Dichtelement (16) in Form eines Dichtelements (16) nach einem der Ansprüche 2 bis 14 ausgebildet ist.

10. Chirurgisches Abdichtungssystem zum Einführen chirurgischer Instrumente in einen menschlichen oder tierischen Körper, vorzugsweise längs einer vom Abdichtungssystem definierten Längsachse (20), umfassend einen eine Einführöffnung aufweisenden Trokar und ein die Einführöffnung mindestens teilweise verschließendes, eine Öffnung (54) aufweisendes chirurgisches Dichtelement (16) zum Abdichten der Einführöffnung beim Einführen eines Instruments in die Öffnung (54), **dadurch gekennzeichnet, dass** das Dichtelement (16) ein Dichtelement (16) nach einem der Ansprüche 1 bis 7 oder eine chirurgische Dichtung nach einem der Ansprüche 8 oder 9 ist.

11. Chirurgisches Abdichtungssystem nach Anspruch 10, **gekennzeichnet durch** eine Schutzvorrichtung (146) für das Dichtelement (16) oder die Dichtung.

12. Chirurgisches Abdichtungssystem nach Anspruch 11, **dadurch gekennzeichnet, dass** die Schutzvorrichtung (146) einen am Trokar oder an einem Teil desselben anordenbaren, ringförmigen oder im Wesentlichen ringförmigen und eine Durchbrechung definierenden Grundkörper (148) mit mehreren in Umfangsrichtung angeordneten und parallel oder auf eine Längsachse (20) der Schutzvorrichtung (146) hin weisenden Schutzelementen (158, 160) umfasst, welche Schutzelemente (158, 160) im Wesentlichen in distaler Richtung weisende freie Enden (162, 164) aufweisen, und dass eine am Grundkörper (148) angeordnete Verbindungseinrichtung zum Verbinden der Schutzvorrichtung (146) mit dem chirurgischen Dichtelement (16) oder dem chirurgischen Abdichtungssystem vorgesehen ist.

13. Chirurgisches Abdichtungssystem nach Anspruch 12, **dadurch gekennzeichnet, dass** in der Grundstellung die Schutzelemente (158, 160) durch das Stützelement (62) in Richtung auf die Längsachse (20) hin vorgespannt sind.

14. Chirurgisches Abdichtungssystem nach einem der Ansprüche 10 bis 13, **gekennzeichnet durch** mindestens einen in Richtung auf die Längsachse (20) oder im Wesentlichen auf die Längsachse hin wirkenden Anschlag (172), welcher das Stützelement (62) in Umfangsrichtung mindestens abschnittsweise umgibt und in der Grundstellung von der das Stützelement (62) von der Längsachse (20) weg weisend begrenzenden Wand (42) beabstandet ist.

15. Chirurgisches Abdichtungssystem nach Anspruch 14, **dadurch gekennzeichnet, dass** der mindestens eine Anschlag (172) an einer Dichtelementhalterung (18) und/oder an einem das Dichtelement (16) oder die chirurgische Dichtung umgebenden Gehäuse angeordnet oder ausgebildet ist.
